# EUROPÄISCHE PATENTANMELDUNG

(11) **EP 1 297 829 A1**
(43) Veröffentlichungstag der Anmeldung: **02.04.2003**
(21) Anmeldenummer: 02020277.6
(22) Anmeldetag: 11.09.2002
(51) Int. Cl.: A61K 31/045, A61K 31/19

(54) **Gegen Bakterien, Mycota und Viren wirksame Wirkstoffkombinationen auf der Basis von mehrwertigen Alkoholen und dialkylsubstituierten Essigsäuren**

(30) Priorität: 26.09.2001 DE 10147545
(71) Anmelder: Beiersdorf AG, 20245 Hamburg (DE)
(72) Erfinder: Traupe, Berns, 22457 Hamburg (DE); Jung, Susanne, 21357 Barddowick (DE); Müller, Martina, 23558 Henstedt-Ulzburg (DE); Maurer, Peter, 22880 Wendel (DE); Oelrichs, Ilka, 25436 Tornesch (DE)

(57) **Zusammenfassung**

Verwendung von Wirkstoffkombinationen, bestehend aus
(I) einem oder mehreren mehrwertigen Alkoholen (Polyolen) sowie
(II) einer oder mehreren dialkylsubstituierten Essigsäuren der Formel 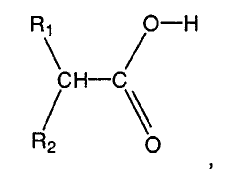
wobei R₁ einen verzweigten oder unverzweigten Alkylrest mit 1 - 12 Kohlenstoffatomen und R₂ einen verzweigten oder unverzweigten Alkylrest mit 1 - 24 Kohlenstoffatomen darstellt,
zur Herstellung kosmetischer oder dermatologischer Zubereitungen gegen Mikroorganismen.

## Beschreibung

Die vorliegende Erfindung betrifft die Verwendung an sich bekannter Substanzen als gegen Bakterien, Mycota und Viren wirksame Substanzen. In besonderen Ausführungsformen betrifft die vorliegende Erfindung kosmetische und dermatologische Zubereitungen, solche Substanzen enthaltend.

Der gesunde warmblütige Organismus, insbesondere die gesunde menschliche Haut, ist mit einer Vielzahl nichtpathogener Mikroorganismen besiedelt. Diese sogenannte Mikroflora der Haut ist nicht nur unschädlich, sie stellt einen wichtigen Schutz zur Abwehropportunistischer oder pathogener Keime dar.

Bakterien gehören zu den prokaryotischen Einzellern. Sie können grob nach ihrer Form (Kugel, Zylinder, gekrümmter Zylinder) sowie nach dem Aufbau ihrer Zellwand (grampositiv, gramnegativ) unterschieden werden. Feinere Unterteilungen tragen auch der Physiologie der Organismen Rechnung. So existieren aerobe, anaerobe sowie fakultativ anaerobe Bakterien. Manche Individuen sind in ihrer Eigenschaft als pathogene Keime von medizinischer Bedeutung, andere wiederum sind vollkommen harmlos.

Gegen Bakterien wirksame Substanzen sind seit geraumer Zeit bekannt. Der Begriff "Antibiotika" beispielsweise, der nicht auf alle antimikrobiell wirksamen Substanzen anwendbar ist, läßt sich auf das Jahr 1941 datieren, obwohl die ersten Erkenntnisse zum Penicillin bereits im Jahre 1929 gefunden wurden. Antibiotika im heutigen Sinne sind nicht für alle medizinischen, schon gar nicht kosmetische Anwendungen geeignet, da häufig auch der warmblütige Organismus, also etwa der erkrankte Patient, bei Anwendung auf irgendeine Weise in seinen Stoffwechselfunktionen beeinträchtigt wird.

Eine Aufgabe der vorliegenden Erfindung war also, den Stand der Technik in dieser Richtung zu bereichern, insbesondere also, Substanzen zur Verfügung zu stellen, welche gegen grampositive und/oder gramnegative Bakterien wirksam sind, ohne daß mit der Anwendung der Substanzen eine unvertretbare Beeinträchtigung der Gesundheit des Anwenders verbunden wäre.

Gramnegative Keime sind beispielsweise Escherichia coli, Pseudomonas-Arten sowie Enterobacteriaceen, wie etwa Citrobacter.

Auch grampositive Keime spielen in Kosmetik und Dermatologie eine Rolle. Bei der unreinen Haut beispielsweise sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Eine besondere Aufgabe der vorliegenden Erfindung war es also, einen gegen unreine Haut bzw. Propionibacterium acnes wirksamen Stoff bzw. Stoffkombination zu finden.

Die vorliegende Erfindung betrifft in einer weiteren Ausführungsform kosmetische Desodorantien. Solche Formulierungen dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann.
Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Mittel, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Eine weitere Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Pilze, auch Fungi [fungus = lat. Pilz], Mycota [µυκηζ= grch. Pilz] oder Mycobionten genannt, zählen im Gegensatze zu den Bakterien zu den Eucaryonten. Eucaryonten sind Lebewesen, deren Zellen (Eucyten) im Gegensatz zu denen der sogenannten Procaryonten (Procyten) über einen durch Kernhülle und Kernmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehltau (Phycomycetes) und die Ständerpilze (Basidiomycetes).

Pilze, auch nicht die Basidiomyceten, sind keine pflanzlichen Organismen, haben aber' wie diese eine Zellwand, zellsaftgefüllte Vakuolen und eine mikroskopisch gut sichtbare Plasmaströmung. Sie enthalten keine photosynthetischen Pigmente und sind C-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophytien (z.B. Epidermophytie, Favus, Mikrosporie, Trichophytie), Hefemycosen (z.B. Pityriasis und andere Pityrosporum-bedingte Mycosen, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose und Skopulariopsidose), Systemmycosen (z.B. Chromomycose, Coccidiomycose, Histoplasmose).

Zu den pathogenen und fakultativ pathogenen Keimen gehören beispielsweise aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen. Eine Beteiligung vonPityrosporum ovale an der Entstehung von Psoriasis wird von der Fachwelt diskutiert.

Alle Bereiche der menschlichen Haut können von Dermatomycosen befallen werden. Dermatophytien befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche.

Ferner sind Superinfektionen der Haut durch Pilze und Bakterien nicht selten.

Bei bestehendem Primärinfekt, d.h., der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten -unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabe-. handlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nenneswerte Einbußen erleidet.

Im Gegensatze zu den prokaryotischen und eukaryotischen zellulären Organismen sind Viren [virus = lat. Gift] biologische Strukturen, welche zur Biosynthese eine Wirtszelle benötigen. Extrazelluläre Viren (auch "Virionen" genannt) bestehen aus einer ein- oder doppelsträngigen Nukleinsäuresequenz (DNS oder RNS) und einem Proteinmantel (Capsid genannt), gegebenenfalls einer zusätzlichen lipidhaltigen Hülle (Envelope) umgeben. Die Gesamtheit aus Nukleinsäure und Capsid wird auch Nucleocapsid genannt. Die Klassifikation der Viren erfolgte klassisch nach klinischen Kriterien, heutzutage allerdings zumeist nach ihrer Struktur, ihrer Morphologie, insbesondere aber nach der Nukleinsäuresequenz.

Medizinisch wichtige Virengattungen sind beispielsweise Influenzaviren (Familie der Orthomyxoviridae), Lyssaviren (z.B. Tollwut, Familie der Rhabdoviren) Enteroviren (z.B. Hepatitis-A, Familie der Picornaviridae), Hepadnaviren (z.B. Hepatitis-B, Familie der Hepadnaviridae).

Viruzide, also Viren abtötende Substanzen im eigentlichen Sinne gibt es nicht, da Viren nicht über eigenen Stoffwechsel verfügen. Es wurde aus diesem Grunde auch diskutiert, ob Viren als Lebewesen eingeordnet werden sollten. Pharmakologische Eingriffe ohne Schädigung der nicht befallenen Zellen ist jedenfalls schwierig. Mögliche Wirkmechanismen im Kampfe gegen die Viren sind in erster Linie die Störung deren Replikation, z.B. durch Blockieren der für die Replikation wichtigen Enzyme, die in der Wirtszelle vorliegen. Ferner kann das Freisetzen der viralen Nukleinsäuren in die Wirtszelle verhindert werden. Im Rahmen der hiermit vorgelegten Offenbarung wird unter Begriffen wie "antiviral" oder "gegen Viren wirksam", "viruzid" oder ähnlichen die Eigenschaft einer Substanz verstanden, einen ein- oder mehrzelligen Organismus vor schädlichen Folgen einer Virusinfektion, sei es prophylaktisch oder therapeutisch, zu schützen, ungeachtet dessen, was der tatsächliche Wirkmechanismus der Substanz im Einzelfalle sei.

Dem Stande der Technik mangelt es jedoch an gegen Viren wirksamen Substanzen, welche zudem den Wirtsorganismus nicht oder nicht in vertretbarem Maße schädigen.

Eine Aufgabe der vorliegenden Erfindung war also, diesem Übelstande abzuhelfen, also Substanzen zu finden, welche wirksam einen ein- oder mehrzelligen Organismus vor schädlichen Folgen einer Virusinfektion, sei es prophylaktisch oder therapeutisch, zu schützen.

Es wurde überraschenderweise gefunden, und darin liegt die Lösung all dieser Aufgaben, daß die Verwendung von Wirkstoffkombinationen, bestehend aus
(I) einem oder mehreren mehrwertigen Alkoholen (Polyolen) sowie
(II) einer oder mehreren dialkylsubstituierten Essigsäuren der Formel 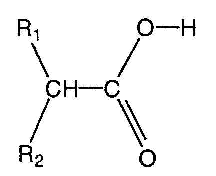 wobei R₁ einen verzweigten oder unverzweigten Alkylrest mit 1 - 12 Kohlenstoffatomen und R₂ einen verzweigten oder unverzweigten Alkylrest mit 1 - 24 Kohlenstoffatomen darstellt,
zur Herstellung kosmetischer oder dermatologischer Zubereitungen gegen Mikroorganismen den Nachteilen des Standes der Technik abhilft.

Vorteilhaft werden die Alkylreste der dialkylsubstituierter Essigsäuren so gewählt daß R1 = Methyl, Ethyl, Propyl, Butyl, Pentyl oder Hexyl darstellt.

Weiter vorteilhaft werden die Alkylreste so gewählt, daß R2= Octyl, Nonyl, Decyl, Undecyl, Dodecyl darstellt.

Insbesondere vorteilhaft ist, die erfindungsgmäßen Essigsäurederivate aus der Gruppe 2-Butyloctansäure, 2-Butyldecansäure, 2-Hexyloctansäure, 2-Hexyldecansäure zu wählen.

Ganz besonders bevorzugt ist die 2-Butyloctansäure.

Es hat sich in erstaunlicher Weise herausgestellt, daß die erfindungsgemäßen Wirkstoffkombinationen das Wachstum von grampositiven und gramnegativen Bakterien, Mycobionten sowie Viren verhindern.

Insbesondere sind die erfindungsgemäßen Wirkstoffkombinationen befähigt, daß Wachstum von Hefen, insbesondere der Pityrosporum-Arten, namentlich Pityrosporum ovale, zu verhindern.

Es hat sich ferner herausgestellt, daß die erfindungsgemäßen Wirkstoffkombinationen die Bildung von seborrhoischen Erscheinungen, insbesondere Kopfschuppen, verhindern sowie bereits vorhandene seborrhoische Erscheinungen, insbesondere Kopfschuppen, zu beseitigen.

Die erfindungsgemäßen Wirkstoffkombinationen eignen sich darüberhinaus gut für die. Verwendung als desodorierender Wirkstoff in kosmetischen Desodorantien sowie gegen unreine Haut, leichte Formen der Akne bzw. Propionibakterium acnes.

Schließlich hat sich herausgestellt, daß die erfindungsgemäßen Wirkstoffkombinationen den Verderb organischer Substanz, insbesondere kosmetischer und dermatologischer Zubereitungen, durch den Befall mit grampositiven und gramnegativen Bakterien, Mycobionten und Viren verhindern können, wenn sie diesen Zubereitungen zugesetzt werden.

Erfindungsgemäß sind somit auch ein Verfahren zur Bekämpfung von Mycobionten, dadurch gekennzeichnet, daß erfindungsgemäße Wirkstoffkombinationen, gegebenenfalls in einem geeigneten kosmetischen oder dermatologischen Träger, mit dem durch Mycobionten kontaminierten Bereich in Kontakt gebracht werden, sowie ein Verfahren zum Schutze organischer Produkte vor dem Befall mit Mycobionten, dadurch gekennzeichnet, daß diesen organischen Produkten dialkylsubstituierte Essigsäuren in wirksamer Menge zugegeben werden.

Der Stand der Technik lieferte folglich nicht den geringsten Hinweis auf die erfindungsgemäße Verwendung als antimycotisches Wirkprinzip.

Ferner war erstaunlich, daß die erfindungsgemäßen Wirkstoffkombinationen besonders gut wirksam sind gegen den für das Entstehen von Kopfschuppen verantwortlichen Keim Pityrosporum ovale und verwandte Keime. Eine bevorzugte Ausführungsform der vorliegenden Erfindung sind mithin gegen Kopfschuppen anzuwendende Formulierungen, beispielsweise Antischuppenshampoos.

Erfindungsgemäß werden die dialkylsubstituierten Essigsäuren bevorzugt in kosmetischen oder dermatologischen Zusammensetzungen eingesetzt einem Gehalt von 0,005 - 50,0 Gew.-%, insbesondere 0,01 - 20,0 Gew.-%, bezogen auf das Gesamtgewicht der Zusammensetzung bevorzugt sind. Vorteilhaft enthalten die Zusammensetzungen 0,02 - 10,0 Gew.-%, besonders bevorzugt 0,02 - 5,0 Gew.-% an den erfindungsgemäßen dialkylsubstituierten Essigsäuren, ganz besonders vorteilhaft 0,5 - 3,0 Gew.-%, jeweils bezogen auf das Gesamtgewicht der Zusammensetzung.

Es ist auch von Vorteil, anstatt reiner erfindungsgemäßer dialkylsubstituierter Essigsäuren solche Stoffe zu verwenden, welche sich ihrerseits durch einen Gehalt an erfindungsgemäßen dialkylsubstituierten Essigsäuren auszeichen.

Die Gesamtmenge an einem oder mehreren Polyolen wird in den fertigen kosmetischen oder dermatologischen Zubereitungen vorteilhaft kleiner als 20,0 Gew.-%, bevorzugt zwischen 1,0 und 15,0 Gew.-% besonders bevorzugt zwischen 2,0 und 10,0 Gew.-%, bezogen auf das Gesamtgewicht der Zubereitungen, gewählt.

Erfindungsgemäß können die Polyole können vorteilhaft gewählt werden aus der Gruppe der mindestens bifunktionellen Alkohole. Vorteilhaft sind insbesondere die Polyole, gewählt aus der folgenden Gruppe:

Ethylenglycol, Polyethylenglycole mit Molmassen bis zu ca. 2.000, Propylenglycol-1,2, Polypropylenglycole-1,2 mit Molmassen bis zu ca. 2.000, Propylenglycol-1,3, Polypropylenglycole-1,3 mit Molmassen bis zu ca. 2.000, Butylenglycol-1,2, Polybutylenglycole-1,2 mit Molmassen bis zu ca. 2.000, Butylenglycol-1,3, Polybutylenglycole-1,3 mit Molmassen bis zu ca. 2.000, Butylenglycol-1,4, Polybutylenglycole-1,4 mit Molmassen bis zu ca. 2.000, Butylenglycol-2,3, Polybutylenglycole-2,3 mit Molmassen bis zu ca. 2.000, Glycerin, Diglycerin, Triglycerin, Tetraglycerin und Pentaglycerin, wobei die Oligoglycerine aus über eine oder mehrere Etherbrücken kondensierten Glycerineinheiten zusammengesetzt sind, beispielsweise wie folgt:

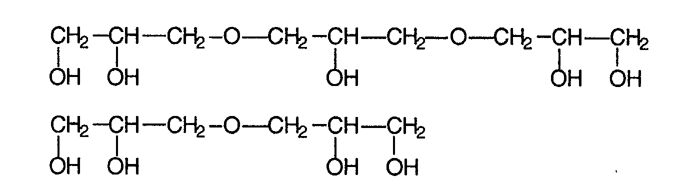

Bevorzugte Polyole sind die monomeren Butylenglycole. Ganz besonders bevorzugt sind 1,2-Propylenglycol und 1,3-Butylenglycol.

Es ist von Vorteil, Gewichtsverhältnisse von dialkylsubstituierten Essigsäuren und Polyolen aus dem Bereich von 1 : 10 bis 10 : 1, bevorzugt 1 : 4 bis 4 : 1, zu wählen.

Erfindungsgemäße Zubereitungen, die erfindungsgemäßen Wirkstoffkombinationen enthaltend, sind besonders vorteilhaft dadurch gekennzeichnet, daß die erfindungsgemäßen Wirkstoffkombinationen in Konzentrationen von 0,05 - 10,00 Gew.-%, bevorzugt 0,1 - 5,0 Gew.-%, vorliegen, jeweils bezogen auf das Gesamtgewicht der Zubereitungen.

Erfindungsgemäße dermatologische Zubereitungen können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, jedoch auch in Form von aus normalen Flaschen und Behältern auftragbaren W/O- oder O/W-Emulsionen, z.B. Crèmes oder Lotionen. Weiterhin können die Zubereitungen vorteilhaft in Form von Tinkturen, Shampoos, Wasch-, Duschoder Badezubereitungen oder Pudern vorliegen.

Als übliche kosmetische Trägerstoffe zur Herstellung der erfindungsgemäßen dermatologischen Zubereitungen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Als Treibmittel für erfindungsgemäße, aus Aerosolbehältern versprühbare dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Als Emulgatoren zur Herstellung der erfindungsgemäßen dermatologischen Zubereitungen, haben sich nichtionogene Typen, wie Polyoxyethylen-Fettalkoholether, z.B. Cetylstearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den erfindungsgemäßen dermatologischen Zubereitungen, deren pH-Wert vorzugsweise z.B. durch übliche Puffergemische auf 4,0 bis 7,5 insbesondere 5,0 bis 6,5, eingestellt wird, Parfüm, Farbstoffe, Antioxidantien, Suspendiermittel, Puffergemische oder andere übliche kosmetische oder dermatologische Grundstoffe beigemischt werden.

Erfindungsgemäß können als günstige Antioxidantien alle für kosmetische und/oder dermatologische Anwendungen geeigneten oder gebräuchlichen Antioxidantien verwendet werden.

Es ist auch von Vorteil, den erfindungsgemäßen Zubereitungen Antioxidantien zuzusetzen. Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist für die vorliegende Erfindung vorteilhaft, daß der pH-Wert der erfindungsgemäßen dermatologischen Zubereitungen kleiner als 8 ist. pH-Werte, die leicht höher sind als 7, aber kleiner als 7,5 können dabei im allgemeinen toleriert werden. Jedenfalls ist für ein gegebenes Fettsäuregemisch im Einzelfalle durch einfaches Ausprobieren, ohne erfinderisches Dazutun, leicht zu ermitteln, welche exakte obere pH-Grenze zu beachten ist.

Vorteilhaft wird der pH-Wert der erfindungsgemäßen Formulierungen im sauren bis sehr schwach alkalischen Bereich kleiner als 8 eingestellt, bevorzugt von 4,0 - 7,5 , besonders bevorzugt von 5,0 - 6,5.

Die jeweils einzusetzenden Mengen an Hilfs- Zusatz- und Trägerstoffen und gegebenenfalls Parfüm können in Abhängigkeit von der Art des jeweiligen Produktes vom Fachmann durch einfaches Ausprobieren leicht ermittelt werden.

Die Herstellung der erfindungsgemäßen dermatologischen Zubereitungen erfolgt, abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen hergestellt und dann emulgiert.

Ansonsten sind die üblichen Maßregeln für das Zusammenstellen von galenischen Formulierungen zu beachten, die dem Fachmann geläufig sind.

Sollen die erfindungsgemäßen Kombinationen in Puder eingearbeitet werden, so können die Suspensionsgrundlagen dafür vorteilhaft gewählt werden aus der Gruppe

Kieselsäuregele (z.B. solche die unter dem Handelsnamen Aerosil® erhältlich sind), Kieselgur, Talkum, modifizierte Stärke, Titandioxid, Seidenpulver, Nylonpulver, Polyethylenpulver und verwandten Stoffen.

Es folgen vorteilhafte Ausführungsbeispiele der vorliegenden Erfindung. Die Mengenangaben beziehen sich stets auf Gewichts-%, wenn nichts Anderes angegeben wird.

### Beispiel 1

### Aerosolspray Typ A:

| | I | II |
|---|---|---|
| 2-Octyldodecanol | 0,50 | 0,50 |
| 1,2-Propylenglykol | 1,00 | 1,00 |
| 2-Butyloctansäure | 0,30 | 0,30 |
| 1,2-Decandiol | - | 0,25 |
| 1,2-Pentandiol | 0,25 | - |
| Parfum | q.s. | q.s. |
| Ethanol | ad 100,00 | ad 100,00 |

Die durch Zusammenmischung der jeweiligen Bestandteile erhaltene flüssige Phase wird mit einem Propan-Butan-Gemisch (2:7) im Verhältnis 39:61 in Aerosolbehälter abgefüllt.

### Beispiel 2

### Aerosolspray Typ B:

| | I | II |
|---|---|---|
| Aluminiumchlorohydrat | 45,00 | 25,00 |
| Isopropylpalmitat | 25,00 | 30,00 |
| Cyclomethicone | ad 100,00 | 0,30 |
| Isoparaffin | - | ad 100,00 |
| Talkum | - | 10,00 |
| 2-Hexyldecansäure | 0,30 | 0,30 |
| 1,2-Octandiol | - | 0,50 |
| 1,2-Decandiol | 0,30 | - |
| Parfum | q.s. | q.s. |

Die durch Zusammenmischung der jeweiligen Bestandteile erhaltene flüssige Phase wird mit einem Propan-Butan-Gemisch (2:7) im Verhältnis 17:83 in Aerosolbehälter abgefüllt.

### Beispiel 3

### Pumpzerstäuber:

| | I | II |
|---|---|---|
| Ethanol | 55,00 | 55,00 |
| PEG-40 Hydriertes Rizinusöl | 2,00 | 2,00 |
| Glycerin | 1,00 | 1,00 |
| 2-Butyloctansäure | 0,40 | 0,40 |
| 1,2-Octandiol | - | 0,30 |
| 1,2-Hexandiol | 0,40 | - |
| Parfum | q.s. | q.s. |
| Wasser | ad 100,00 | ad 100,00 |

### Beispiel 4

### Roll-on Gel:

| | I | II |
|---|---|---|
| Ethanol | 50,00 | 50,00 |
| PEG-40 Hydriertes Rizinusöl | 2,00 | 2,00 |
| Hydroxyethylcellulose | 0,50 | 0,50 |
| 2-Butyloctansäure | 0,25 | 0,35 |
| 1,2-Decandiol | - | 0,20 |
| 1,2-Hexadecandiol | 0,25 | - |
| Aluminiumchlorohydrat | - | 10,00 |
| Parfum | q.s. | q.s. |
| Wasser | ad 100,00 | ad 100,00 |

### Beispiel 5

### Roll-on Emulsion:

| | I | II |
|---|---|---|
| Aluminiumchlorohydrat | - | 10,00 |
| Polypropylenglykol(15)stearylether | 5,00 | 5,00 |
| Polyethylenglykol(100)stearylether | 1,00 | 1,00 |
| Polyethylenglykol(2)stearylether | 4,00 | 4,00 |
| 2-Hexyldecansäure | 0,50 | 0,50 |
| 1,2-Hexandiol | 0,20 | - |
| 1,2-Dodecandiol | - | 0,30 |
| Parfum, Konservierungsstoffe | q.s. | q.s. |
| Wasser | ad 100,00 | ad 100,00 |

### Beispiel 6

### Aerosol-Rasierschaum:

| | I | II |
|---|---|---|
| Stearinsäure | 6,00 | 6,00 |
| Myristinsäure | 1,00 | 1,00 |
| Polyethylenglykol(20)cetylether | 2,00 | 2,00 |
| Glycerin | 5,00 | 5,00 |
| Triethanolamin | 4,00 | 4,00 |
| 2-Butyloctansäure | 0,25 | 0,35 |
| 1,2-Decandiol | - | 0,35 |
| 1,2-Octadecandiol | 0,75 | - |
| Parfum, Konservierungsstoffe | q.s. | q.s. |
| Wasser | ad 100,00 | ad 100,00 |

Die durch Zusammenmischung der jeweiligen Bestandteile erhaltene flüssige Phase wird mit einem Propan-Isobutan-Gemisch (2:7) im Verhältnis 3:97 in Aerosolbehälter abgefüllt.

### Beispiel 7

### Aerosol-Rasiergel:

| | I | II |
|---|---|---|
| Palmitininsäure | 7,00 | 7,00 |
| Stearinsäure | 1,50 | 1,50 |
| Polyethylenglykol(25)cetearylether | 2,00 | 2,00 |
| Sorbitol | 5,50 | 5,50 |
| Triethanolamin | 5,00 | 5,00 |
| Hydroxyethylcellulose | 0,30 | 0,30 |
| 2-Butyloctansäure | 0,50 | 0,50 |
| 1,2-Decandiol | - | 0,30 |
| 1,2-Pentandiol | 0,50 | - |
| Parfum, Konservierungsstoffe | q.s. | q.s. |
| Wasser | ad 100,00 | ad 100,00 |

Die durch Zusammenmischung der jeweiligen Bestandteile erhaltene flüssige Phase wird mit einem n-Pentan-n-Butan-Gemisch (5:1) im Verhältnis 6:94 in Aerosolbehälter abgefüllt.

### Beispiel 8

### Abdeckstift:

| | I | II |
|---|---|---|
| Paraffinöl | 30,00 | 25,00 |
| Mikrokristallines Wachs | 35,00 | 35,00 |
| Cyclomethicone | 5,00 | 10,00 |
| Talkum | 5,00 | 5,00 |
| Zinkoxid | 5,00 | 5,00 |
| Eisenoxid | 0,80 | 0,80 |
| 2-Butyloctansäure | 0,40 | 0,50 |
| 1,2-Decandiol | - | 0,50 |
| 1,2-Tetradecandiol | 0,80 | - |
| Parfum, Konservierungsstoffe | q.s. | q.s. |
| 2-Octyldodecanol | ad 100,00 | ad 100,00 |

## Patentansprüche

1. Verwendung von Wirkstoffkombinationen, bestehend aus
(I) einem oder mehreren mehrwertigen Alkoholen (Polyolen) sowie
(II) einer oder mehreren dialkylsubstituierten Essigsäuren der Formel 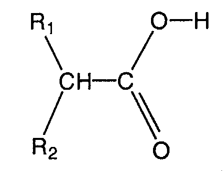 wobei R₁ einen verzweigten oder unverzweigten Alkylrest mit 1 - 12 Kohlenstoffatomen und R₂ einen verzweigten oder unverzweigten Alkylrest mit 1 - 24 Kohlenstoffatomen darstellt,
zur Herstellung kosmetischer oder dermatologischer Zubereitungen gegen Mikroorganismen.

2. Verwendung nach Anspruch 1, **dadurch gekennzeichnet, daß** das oder die Polyole gewählt werden aus der Gruppe Ethylenglycol, Polyethylenglycole mit Molmassen bis zu ca. 2.000, Propylenglycol-1,2, Polypropylenglycole-1,2 mit Molmassen bis zu ca. 2.000, Propylenglycol-1,3, Polypropylenglycole-1,3 mit Molmassen bis zu ca. 2.000, Butylenglycol-1,2, Polybutylenglycole-1,2 mit Molmassen bis zu ca. 2.000, Butylenglycol-1,3, Polybutylenglycole-1,3 mit Molmassen bis zu ca. 2.000, Butylenglycol-1,4, Polybutylenglycole-1,4 mit Molmassen bis zu ca. 2.000, Butylenglycol-2,3, Polybutylenglycole-2,3 mit Molmassen bis zu ca. 2.000, Glycerin, Diglycerin, Triglycerin, Tetraglycerin und Pentaglycerin.

3. Verwendung nach Anspruch 1, **dadurch gekennzeichnet, daß** als dialkylsubstituierte Essigsäure die 2-Butyloctansäure gewählt wird.

4. Verwendung nach Anspruch 1, **dadurch gekennzeichnet, daß** die dialkylsubstituierten Essigsäuren in kosmetischen oder dermatologischen Zusammensetzungen eingesetzt werden, einem Gehalt von 0,005 - 50,0 Gew.-%, insbesondere 0,01 - 20,0 Gew.-%, bevorzugt 0,02 - 10,0 Gew.-%, besonders bevorzugt 0,02 - 5,0 Gew.-%, ganz besonders bevorzugt 0,5 - 3,0 Gew.-%, entsprechend, bezogen auf das Gesamtgewicht der Zusammensetzung.

5. Verwendung nach Anspruch 1, **dadurch gekennzeichnet, daß** die Gesamtmenge an einem oder mehreren Polyolen in den kosmetischen oder dermatologischen Zubereitungen kleiner als 20,0 Gew.-%, bevorzugt zwischen 1,0 und 15,0 Gew.-% besonders bevorzugt zwischen 2,0 und 10,0 Gew.-%, bezogen auf das Gesamtgewicht der Zubereitungen, gewählt wird.

6. Verwendung nach Anspruch 1 zur Bekämpfung von Körpergerüchen.

7. Verwendung nach Anspruch 1 zur Bekämpfung von Kopfschuppen.
